**Europäisches Patentamt**

**European Patent Office**

**Office européen des brevets**

(11) Publication number: **0 133 833**
**A1**

(12) # EUROPEAN PATENT APPLICATION

(21) Application number: **84401612.1**

(22) Date of filing: **01.08.84**

(51) Int. Cl.⁴: **A 61 K 39/385**
**A 61 K 39/29, A 61 K 39/02**
**A 61 K 39/12**

(30) Priority: **01.08.83 FR 8312660**

(43) Date of publication of application:
**06.03.85 Bulletin 85/10**

(84) Designated Contracting States:
**AT BE CH DE FR GB IT LI LU NL SE**

(71) Applicant: **ANVAR Agence Nationale de Valorisation de la Recherche**
**43, rue Caumartin**
**F-75436 Paris Cédex 09(FR)**

(72) Inventor: **Tartar, André**
**rue du Moulin**
**F-62490 Vitry-en-Artois(FR)**

(72) Inventor: **Gras-Masse, Hélène**
**31, rue Petit Flot**
**F-59510 Hem(FR)**

(72) Inventor: **Lefrancier, Pierre**
**46, Allée de la Mare l'Oiseau**
**F-91190 Gif-sur-Yvette(FR)**

(72) Inventor: **Level, Michel**
**24-26, rue Sibuet**
**F-75012 Paris(FR)**

(72) Inventor: **Audibert, Françoise**
**44, rue Ybry**
**F-92200 Neuilly-sur-Seine(FR)**

(72) Inventor: **Chedid, Louis**
**16,18, rue Gaston de Caillavet**
**F-75015 Paris(FR)**

(72) Inventor: **Jolivet, Michel**
**6, rue Edouard Branly**
**F-92130 Issy-les-Moulineaux(FR)**

(72) Inventor: **Beachey, Edwin H.**
**1030 Jefferson Avenue**
**Memphis Tennessee 38104(US)**

(74) Representative: **Gutmann, Ernest et al,**
**Cabinet Plasseraud 84, rue d'Amsterdam**
**F-75009 Paris(FR)**

(54) **Compositions of vaccines conditioned for the vaccination of immunitarily non-naive subjects against a predetermined pathogenic agent and containing a haptene itself comprising an antigenic site characteristic of said pathogenic agent or an oligomer of this haptene.**

(57) The invention relates to a vaccine composition against a predetermined pathogenic agent suitable for the reimmunization of an immunitarily non-naive host. It contains a synthetic haptene possessing itself an antigenic site characteristic of said pathogenic agent or an oligomer of said haptene. It is conditioned in dose-units containing a dosage of haptene adjusted for said re-immunization of the immunitarily non-naive subject, in association with a physiologically acceptable vehicle.

COMPOSITIONS OF VACCINES CONDITIONED FOR THE VACCINATION OF IMMUNITARILY NON-NAIVE SUBJECTS AGAINST A PREDETERMINED PATHOGENIC AGENT AND CONTAINING A HAPTENE ITSELF COMPRISING AN ANTIGENIC SITE CHARACTERISTIC OF SAID PATHOGENIC AGENT OR AN OLIGOMER OF THIS HAPTENE

BACKGROUND OF THE INVENTION

The invention relates to a composition of vaccine conditioned for the vaccination of immunitarily non-naive subjects against a predetermined pathogenic agent and containing a haptene including itself an antigenic site characteristic of said pathogenic agent.

It is known that a certain number of synthetic immunogens have been proposed or even produced. Generally it has already been suggested to substitute for these natural antigens used in human or veterinary vaccination synthetic antigens possessing structural elements in common with these natural or "native" antigens. These structural elements contain in general an antigenic site or "epitope" of the natural antigen. For example, this synthetic antigen contains a peptide sequence in common with the natural or "native" antigen, when the latter consists of a protein; or it may contain an oligosaccharide sequence when the natural antigen possesses a polysaccharide structure. In most instances however synthetic peptides or oligosaccharides limited to these sequences - i.e. the corresponding haptenes, though recognizable by antibodies with respect to the natural antigen, exhibit themselves, not more than an extremely low immunogenic activity _in vivo_, as detectable by the conventional immunological techniques. In addition whether such immunogenicity, even if achieved to some extent, is likely to confer to the host an effective protection against the natural pathogenic agent containing a same antigenic site or determinant is still an open question.

Various methods have been proposed in the literature to reinforce this immunogenicity. The most conventional method consists of associating the haptene with immunological adjuvants, such as the synthetic adjuvants known under the name of "muramyl-peptides".

It has also been proposed to "oligomerize" the haptenes. The latter operation resulted in some cases in an increase of the immunogenic capacity, if any, of the haptene.

Finally, a method currently studied consists in the conjugation of the haptene or of the haptene polymer with a physiologically acceptable carrier-molecule of sufficiently high molecular weight. This conjugation can in fact lead to an appreciable increase in the immunogenicity which can be induced in vitro.

The use of such carrier molecules such as tetanus anatoxin or diphtheria anatoxin, is however difficult, to the extent that they often themselves show considerable immunogenic properties. It seems that they cannot be used for repeated vaccinations, and this all the more as in some cases an irreversible immunosuppression may be observed.

All of these observations must however be contemplated within the real context of immunization tests practiced hitherto with such synthetic vaccines. In fact, these tests have in general been carried out on immunitarily "naive" animals, in that the latter had never previously been exposed, either naturally or not, to the corresponding natural antigens.

GENERAL DESCRIPTION OF THE INVENTION

The invention arises form the discovery that haptenes or haptene oligomers including an epitope of the natural antigen were in fact capable, when they were conditioned at a suitable dosage, of ensuring re-immunization of a subject against the natural antigen, when the subject had already previously been exposed to the natural antigen, in other words the subject was no longer immunitarily naive.

The invention relates accordingly to a vaccine composition against a predetermined pathogenic agent containing a free synthetic haptene itself possessing an antigenic site characteristic of said pathogenic agent or an oligomer, said hapten or oligomer, free of carrier molecules coupled thereto, being conditioned in dosage unit forms suitable for re-immunizing an immunitarily non-naive subject against said pathogenic agent, in association with a physiologically acceptable vehicle.

The invention is thus more particularly concerned with secondary or booster vaccinations in subjects who (or which) in the case of animals despite their immunitarily non-naive character, are no longer suitably protected against the pathogenic antigen. It is then observed that the dose of haptene necessary to obtain re-immunization (secondary vaccination) of the subject who had already previously been subjected to the action of the pathogenic or of the natural vaccinating antigen, is not commensurable with a dose which in theory would be necessary to confer on the subject a similar level of immunity in a primary vaccination. Such a theoretical comparison can moreover could even only be thought of if the haptene showed a minimum effective immunogenicity. The results to which the invention leads, can be interpreted as meaning that it suffices, for recreating in the host's organism the conditions of protection against a pathogenic antigen to which it had already been exposed, in a secondary vaccination to administer a principle including only part of the immunological features of the active principle which were involved in the initial immunitary reaction or primary vaccination against this antigen. This finding appears applicable to all possible situations of prior exposure of a subject to a strongly immunogenic antigen corresponding to the pathogenic agent, whether the latter consisted of the pathogenic agent or of a corresponding strongly immunogenic vaccine composition, for instance a live - attenuated or killed microorganism or an anatoxine.

The invention must enable at least in part the overcoming of numerous difficulties encountered in the course of vaccinations with conventional natural vaccines. As a matter of fact, the risks which can be associated with vaccination appear more at the level of what can in all cases be termed as a "secondary vaccination". The reaction against a primary vaccination is often rather weak. On the other hand, interfering reactions, for example allergies which can be dangerous, are often manifested on the occasion of a secondary vaccination.

The invention hence also relates to a vaccination of immunitarily naive subjects, comprising a primary vaccination effected with a conventional vaccine such as those used hitherto (or with a natural antigen in the sense used in the present description) and at least one secondary vaccination under conditions which can then be much better controlled, with a haptene corresponding to this antigen.

The invention thus enables the use of carrier molecules or "carriers" to be avoided, in contrast with the requirements deemed necessary by most of the authors concerned with the vaccination with synthetic vaccines. The invention thus aims at avoiding the toxic phenomena which could be generated by these carrier molecules, as well as effects which are likely to be tied to the reinforcement of the antigenic characteristics of the hapten-carrier conjugates as a whole.

In the case of a secondary booster vaccination with the unconjugated haptenes, it may even be unnecessary to use an immunological vaccine-adjuvant.

The invention relates therefore also to a novel combination, particularly a kit, suitable for the carrying out of a complete cycle of vaccinations against a determined pathogenic agent, such kit comprising :
- a dose of a vaccinating principle, particularly a natural vaccine, conditioned at a dosage capable of confering a first protective immunization in a naive host, and
- at least one dose for at least one booster vaccination of a haptene containing an antigenic site characteristic of the active principle of the vaccine for the first immunization against the pathogenic agent concerned, or of an oligomer of this haptene, said hapten or oligomer being conditionned in dosage unit forms capable of maintaining or boostering the immunity acquired by the host subsequent to the abovesaid first protective immunization.

The invention is also applicable to hosts who (or which) had already been vaccinated in the past or who (or which) had been exposed in the past to the pathogenic agent, and in whom (or which) the acquired protective immunization needs be evoked again. It is known that under such circumstances, the use of conventional vaccination techniques, with a "natural" vaccine, can generate troublesome side reactions, by reason of the too violent immunitary reactions or of secondary effects, for example, of the allergic type, which can be elicited.

In order to take care of such circumstances the invention also provides a kit for at least one booster vaccination against a predetermined pathogenic agent, containing, in combination :

- means for taking a sample of blood or of serum from the subject to be re-vaccinated;

- the elements or reagents or both suitable for the in vitro diagnosis of the immunitary non-naiveness of the host to be vaccinated against said pathogenic agent on said blood or serum sample;

- control compositions, particularly of blood or serum samples from immunitarily naive and, if need be, non-naive hosts or, alternatively, charts, or both;

- at least one dose of a haptene or of an oligomer of said hapten, said hapten or oligomer containing an antigenic site characteristic of the corresponding pathogenic agent or of a natural vaccine principle active against this pathogenic agent, said haptene or oligomer being conditioned in dosage unit forms effective to maintain or reevoke a past protective immunization against said pathogenic agent.

For example, the elements necessary for effecting said diagnosis on the blood or plasma sample of the host to be revaccinated, comprise a microplate and the reagents necessary for carrying out comparative tests of the antibody levels contained in the blood or serum sample.

Advantageously, the above-indicated comparative type tests are carried out by a method of the ELISA type. In particular, this method will comprise the following steps standardizable according to the natural antigen concerned:

- incubating increasing dilutions of the serum under study and of the control serum in the cups of the microplate, coated with the active principle of the natural vaccine, for a period enabling an optimum reaction (the antibodies to be detected in the serum being hereafter termed as "first antibodies") ;

- after thorough washing of the microplate with a suitable buffer, contacting the first antibodies, if any, fixed in the cups with a second category of antibodies directed against the first, the antibodies of the second category being labelled, preferably by an enzyme selected preferably from among those whose action with respect to the appropriate substrate is manifested by a measurable variation in absorbance of an appropriate wave length therethrough;

- determining the relative levels of antibodies of the second category fixed in the cups relative to the sample under study and in those relative to controls;

- comparing the levels measured, particularly of the absorbancies measured by means of a photometer, in the cups corresponding respectively to the samples under study and to the control sample.

The determination of the limits which are to be used for evaluating the naive or non-naive character of the host under study, according to the results of the above-indicated comparative measurements, can be estimated by the specialist, having regard to the nature of the pathogenic agent concerned.

By way of non-limiting indication, it will be considered that the subject or host under study was not immunitarily naive when the optical densities measured by the preceding technique on serums diluted to 1/100 were higher than at least three times the optimum optical density measured under the same conditions on a serum of a naive host or subject at the same dilution. In particular, it will be considered that the individual under study was not immunitarily naive when the measured optical density on its serum was at least equal to three times the optical density measured on a serum from an immunitarily naive individual, when the technique is put into operation comprising the following successive steps :

- depositing 0.5 to 2 micrograms of the antigen concerned in the cups of the plate;

- adding suitable dilutions, on the one hand, of the serum under study and, on the other hand, of the control serum (except when the values of the corresponding optical densities have been predetermined and are available from charts);

- incubating the plate for the time necessary for reaction, particularly for two hours at 37°C;

- washing the plate with suitable solvents to remove the unfixed proteins or immunoglobulins;

- contacting the plate with antibodies labelled with peroxidase, these antibodies belonging to the abovesaid second category and, after suitable washings of the plate;

- contacting the plate with a solution of 50 mg of O-phenylene-diamine as substrate in 100 ml of a buffer solution 0.05 M citrate/phosphate, pH 5, and containing 20 microliters of 130 volume hydrogen peroxide;

- interrupting the contact after the time necessary for the reaction, particularly from 5 to 30 minutes according to the nature of the antigen used, by the addition of sulfuric acid,

- carrying out optical density measurements and comparing the results with those obtained under the same conditions on the control samples (or with predetermined values available from a chart).

The invention can be practiced with any haptene which can be recognized by an antibody previously formed with respect to a natural vaccinating antigen and possessing at least one epitope in common with this haptene. These haptenes can be exclusively peptidic or non-exclusively peptidic, i.E. a glycoprotein. It may also consist, for example, of an oside structure of low molecular weight, which can be constituted from a monosaccharide or from several monosaccharides linked together, for example by a glycosidic-type linkage. These oligosaccharides may be either linear, or branched. They may belong to the family of hexoses or of pentoses; they may be neutral or have an alkaline or acidic character. For instance they may include an uronic acid or a sialic acid or belong to similar series. They can carry substituents usually contained in natural structures, such as acetyl, sulfate or

0133833

phosphate groups. These indications are merely given by way of example. The haptenes may consist of peptidic or osidic haptenes the structure of which either recur within the structure of an antigen of high molecular weight or not, i.e. in the case of an antigen including a chain, whether the haptenes is included in the chain or is located at a terminal position thereof.

Generally the haptenes which can be used either as such or in oligomer state, for the constitution of vaccine doses usable for a secondary immunization, have a structural element in common, under the conditions which have been mentioned above, for example with active principles extracted from or produced by infectious micro-organisms or the infectious micro-organisms themselves (attenuated or killed vaccines). More particularly, the haptenes which can be employed within the scope of the invention can have structural elements in common with constituents of these pathogenic agents (such as bacteria, viruses, parasites, rickettsias, protozoa, etc.). By way of additional examples, will be mentioned haptenes having structural elements in common with antigenic proteins belonging to the envelopes of viruses of viral B hepatitis, influenza, herpes viruses, or again proteins or bacterial walls, for example, streptococci, etc.. Oligosaccharides may also be m<entioned by way of haptenes having structural elements in common with antigens non exclusively peptidic or non-peptidic antigens. More generally, reference can also be made to the indications provided in European patent application Nr. 3833, which is incorporated herein by reference and particularly as concerns antigens from which haptenes in accordance with this invention can be derived.

The invention relates more particularly, by way of examples, to kits comprising, for a primary vaccination, active principles extracted from corresponding infectious micro-organisms or the infectious micro-organisms themselves (attentuated or killed vaccines) and, for at least one booster vaccination, the haptene or oligomer of the corresponding haptene, or both, particularly when several booster administrations or shots are contemplated.

Among the haptenes which offer great interest within the scope of the application according to the invention are mentioned those which bear an epitope of the surface antigen (HBs) of virus of hepatitis B. Reference can be made, for example, to the peptides described in European patent application Nr. 44 710 of SCRIPPS CLINIC AND RESEARCH FOUNDATION (invention of LERNER et coll.). Mention can also again be made of the very recent publication of John L. GERIN et coll. (Proc. Natl. Acad. Sci. U.S.A. vol. 80 pp. 2,365-2,369, April 1983), as regards the peptides possibly bearing an epitope characteristic of the HBs antigen. It is also possible to resort to the peptide containing at the most 30 amino acid residues and characterized in that it contains the sequence:

Asp - Tyr - Gln - Gly - Met - Leu - Pro - Val - X - Pro - Leu - Ile - Pro - Gly - Ser - Y - Thr - Thr - Ser - Thr - Gly - Z - X

in which:

- X is cysteyl, aminobutyryl, alanyl or seryl,
- Y is seryl or threonyl and
- Z is prolyl or seryl.

Advantageously X is a cysteyl group.

The various representatives of this class of peptides can be synthesized by any technique known in itself, for example by the technique described by R. D. MERRIFIELD in the article entitled "Solid Phase Peptide Synthesis" (J. Am. Chem. Soc., 45, 2149-2154). There will be mentioned in addition synthetic peptides of the type described by E.H. BEACHEY et al which are capable, when coupled with a muramyl-peptide, of forming antibodies against the protein M of the surface of <u>Streptococcus pyogenes.</u> This protein is denoted below, in example II, under the abbreviation "M24". It is also possible to refer to the peptide sequences defined in United States patent Nr. 4,284,537, more particularly those defined under the abbreviations $CB_6$ and $CB_7$. In the example II below the haptene employed is constituted by the peptide S-CB7, which also contains an antigenic site of the protein M 24. The peptide sequence of S-CB7 is as follows:

Asn-Phe-Ser-Thr-Ala-Asp-Ser-Ala-Lys-

Ile-Lys-Thr-Leu-Glu-Ala-Glu-Lys-Ala-Ala-

Leu-Ala-ala-Arg-Lys-Ala-Asp-Leu-Glu-Lys-
Ala-Leu-Glu-Gly-Ala-Met.

Recourse can also be had to one of the following haptenes:

Ala-Ala-Leu-Ala-Ala-arg-Lys-Ala-Leu-Glu-Lys-Gly-
Gly-Gly-

or

Lys-Ala-Asp-Leu-Glu-Lys-Ala-Leu-Glu-Gly-Ala-Met-

or

Asn-Phe-Ser-Thr-Ala-Asp-Ser-Ala-Lys-
Ile-Lys-Thr-Leu-Glu-Ala-Glu-Lys-ala-Ala-
Leu-Ala-Ala-Arg-.

By way of saccharide or oside haptenes will be mentioned for example polysaccharide C of Streptococcus C, cellobiuronic acid (Pneumo type III), as described by W.F. GOEBEL, "J. Exp. Medicine", 1939, pp. 353-363, or para-aminophenyl-cellobiuronic acid. Other preferred haptenes consist of peptide sequences contained in the diphteria toxin, in polypeptides having vaccinating properties against paludism, etc..

Advantageously the booster vaccination is conducted with water-soluble oligomers of the above-indicated monomeric haptene. These oligomers can possibly contain from 2 to 10 monomeric units, although such numerical indications have no limitative significance.

It is possible to resort, to effect oligomerization, to any polymerisation technique currently used in the field of peptides, this polymerisation being conducted until an oligomer or polymer is obtained containing the number of monomeric units required for acquiring the desired immunogenicity.

A preferred method of oligomerization or polymerisation of the monomer consists of the reaction of the latter with a cross-linking agent such as glutaraldehyde.

It is also possible to resort to other oligomerization or coupling methods, for example those employing successive couplings of monomeric units, through their terminal carboxyl and amino functions or other reactive groups, for example -SH, in the presence of homo- or hetero-bifunctional coupling agents.

DESCRIPTION OF PREFERRED EMBODIMENTS

Additional features of the invention will also appear in the course of the description which follows of preferred embodiments of the invention, given of course purely by way of non-limiting examples.

EXAMPLE I

Secondary immunization with a peptide containing an epitope of HBs antigen.

a) Synthesis of the peptide of the formula (called "peptide 99-121")

Asp-Tyr-Gln-Gly-Met-Leu-Pro-Val-Cys-Pro-Leu-Ile-
100                                           110
Pro-Gly-Ser-Ser-Thr-Thr-Ser-Thr-Gly-Pro-Cys
                        120

The synthesis was carried out according to the solid phase technique of MERRIFIELD, by means of an automatized synthesizer of the BECKMANN 990 B type.

The peptide chain was prepared from the first C-terminal aminoacid, fixed covalently on a polymer of styrene and divinylbenzene (which can also be of the polyamide type) by a linkage of the benzyl ester type (chloromethylated resin) or amide type (benzhydrylamine resin). The following aminoacids, towards the N-terminal portion were added successively by repetition of the cycle of operations indicated in Table 1. The principal steps thereof included the steps mentioned hereafter are :

1) Starting from aminoacids or peptides whose N-terminal groups are protected by a tertio-butyloxycarbonyl group (BOC) deprotection of the amino groups by means of a solution of trifluoroacetic acid (TFA) in $CH_2Cl_2$.

2) Neutralization of the amino functions by means of a solution of diisopropylamine (D I E A) in $CH_2Cl_2$.

3) Activating the carboxyl groups of the aminoacid to be linked to the aminoacid or peptide hereabove by means of dicyclohexyl-carbodiimide or by activated ester method (for instance with orthonitrophenol). The various coupling reagents are added in excess (3 to 6 times) with respect to the amount used of the resin. After each coupling, the absence of free amino group on the resin is checked by means of the ninhydrin test. In case the

0133833

reaction is positive, the coupling is repeated.

In the course of the synthesis the reactive groups borne by the side chains of the aminoacids are protected by means of groups figuring in Table 2.

At the end of synthesis, the peptide was released from the resin and from its protective groups by treatment for one hour with anhydrous hydrofluoric acid containing 10 % of anisol (V/V). Only the acetamidomethyl (acm) group protecting the cystein remained intact at the end of this treatment. The peptide was then reduced by means of di-thiothreitol in a buffer at pH8, then purified by gel filtration and reverse phase partition chromatography.

Its identity was checked by aminoacid analysis of the acid hydrolysis and its homogeneity by chromatography on a thin silica layer in three different solvent systems as well as by reverse phase high pressure liquid chromatography.

TABLE I

SYNTHESIS PROCEDURE

| Reagent | Volume per 1 g of resin | Number of operations | Time |
|---|---|---|---|
| $CH_2Cl_2$ | 15 ml | x 3 | 2 min |
| TFA - $CH_2Cl_2$ (4/6) | 13 ml | x 1 | 1 min |
| TFA - $CH_2Cl_2$ (2/3) | 13 ml | x 1 | 30 min |
| $CH_2Cl_2$ | 13 ml | x 5 | 2 min |
| DIEA - $CH_2Cl_2$ (1/20) | 15 ml | x 3 | 2 min |
| $CH_2Cl_2$ | 15 ml | x 5 | 2 min |
| coupling | 15 ml | x 1 | 90 min |
| $CH_2Cl_2$ | 15 ml | x 5 | 2 min |

Groups protecting side chains

TABLE II

| Aminoacid | Position | Protective group |
|-----------|----------|------------------|
| Asp | 99 | Benzyl ester |
| Tyr | 100 | O-dichlorobenzyl |
| Cys | 107 | S-acetamidomethyl |
| Ser | 113-114-117 | O-benzyl |
| Thr | 115-116-118 | O-benzyl |
| Cys | 121 | S-p-methoxybenzyl |

b) Obtaining a secondary response with peptide 99-121
   immunization with the natural HBsAg antigen (Table I)

Mice (15) had received subcutaneously on Day 1, the natural antigen with $Al(OH)_3$ used as an adjuvant. After 1 month, each initial batch was divided into three:

(a) 5 mice received nothing,

(b) 5 mice received a natural antigen booster and

(c) 5 others the synthetic antigen.

The serums were collected after 8 days.

The serums were titrated by passive hemagglutination. The figures indicate the greatest dilution of serum capable of agglutinating human blood cells sensitized to HBs antigen ("National Center of Blood Transfusion" of France).

The results are shown in Table III which follows. It shows clearly the "booster" effect exerted by the synthetic peptide with respect to blood immunogenicity, subsequent to the primary immunization carried out with the natural HBs antigen.

- 15 -                    0133833

## TABLE III

| Immunization | | Anti-HBs |
| Primary<br>J 1 | Secondary<br>J 30 | antibody titers<br>on Day 38 |
|---|---|---|
| HBs 1 $\mu$g+Al(OH)$_3$ 40 $\mu$g | (a) – | 512 |
| | (b) HBs 1 $\mu$g | 8192 |
| | (c) (99-121) 100 $\mu$g | 2048 |

EXAMPLE II

Secondary vaccination with an oligomer of the peptide SCB-7 against streptococcal infections.

a) Preparation of the oligomer

15 mg of the abovesaid peptide (disclosed by BEACHEY et al, 1981, Nature, 292, 457-459) was dissolved in a 0.1 M sodium bicarbonate solution (3 ml). The solution was mixed with an aqueous soluton of 250 g of glutaraldehyde/liter to obtain the final concentration of 0.01 % of glutaraldehyde. The reaction was carried out at ambiant temperature, in darkness and with stirring. The reaction was allowed to continue for three days. The oligomer formed was then dialysed against a suitable buffer, such as PBS.

It is this oligomer denoted under the expression "oligo-SCB-7" which was used in the vaccination sequence which follows.

b) Booster immunization against streptococcal infections with SCB-7 and oligo-SCB-7

Two groups of four mice received at Day 0 and at Day 60 a primary injection of 10 micrograms of protein M-24 (disclosed in US patent Nr. 4,284,537). One of the groups then received on Day 115, then on Day 200, the doses indicated in the table of peptide SCB-7 and oligo-SCB-7. In all cases, the immunogenic agents were administered in the form of a solution in PBS solvent, in the absence of any adjuvant.

The animals of the other group received on Day 115 and on Day 200 the PBS solution, devoid of the immunogenic agent.

0133833

The levels of anti-SCB-7 and anti-M-24 antibodies respectively indicated in each of the treated animals are indicated in Table IV below. The mean values of the antibody titers obtained with the control group are also indicated. The antibody titers were measured by a technique derived from the ELISA method carried out under the experimental conditions described by AUDIBERT, F. et al, Proc. Natl. Acad. Sci. USA, 79, 5042-5046.

In particular, doses of the protein M-24 and of the peptide SCB-7 were deposited in each of the cups of a NUNC micro-titration plate, in the proportions of 0.4 micrograms of the M-24 protein and of 2 micrograms of peptide SCB-7, respectively in the appropriate assays. The plates were placed under incubation for 2 hours at 37°C. After five washings with a buffer solution, the plates were incubated again for 1 hour with a serum containing anti-sheep goat immunoglobulins labelled with peroxidase. After thorough washing of the plates, there were added to the cups a substrate solution (O-phenylene-diamine) in a 0.05 M citrate/phosphate buffer, pH 5 (50 mg of substrate/100 ml of buffer) and 20 microliters of a 130 volume hydrogen peroxide solution. The reaction was allowed to proceed for 10 minutes as regards the tests carried out in the cups in which the protein M-24 had been deposited and for 7 minutes as regards those cups in which the peptide SCB-7 had been deposited. The reactions were interrupted by the addition of 12.5 % sulfuric acid. The absorbances of the 492 nm radiation were then read in an ELISA reader of the "Multiskan Titerteck" type marketed by "Flow Laboratories". Similar tests were carried out with a normal mouse serum as a negative control.

The titers of antibodies measured at Day 100, that is to say before the first of the booster injections, were very low, as emerges from Table IV.

On the contrary, significant immunogenic responses were observed on Day 225, hence after a first booster injection of SCB-7 and a second booster injection of oligo SCB-7, with respect both to the peptide SCB-7 and the oligo SCB-7. These results are naturally to be compared with those obtained in control animals, whose mean titers measured at Day 225 are also indicated in the Table.

These results are particularly interesting, to the extent that they have been obtained by administration of the antigens in a saline solution in the absence of any adjuvant. They authorized effective vaccination of man to be envisaged against streptococcal infections directly with the oligopeptide, to the extent that in most instances humans are no longer immunitarily naive. They either already underwent a vaccination or had already been exposed to an infection with streptococci.

The antibodies obtained after a booster with the oligo SCB-7 are protective; this has been shown in the opsonophagocytosis test described by BEACHEY et coll. (JEM, 1979, 150, 862). The experimental serums have permitted the phagocytosis of streptococci of a homologous type by human neutrophils.

Moreover the oligopeptides bearing an antigenic site of the M-24 protein (or the antigenic site contained in SCB-7) seem all the more favorable as they seem devoid of side reactions which can be induced in subjects treated with the M-24 protein, especially when the latter is used in booster shots. These side reactions are capable of being manifested in the form of cardiac pathology. In particular, serums from treated animals as indicated above do not react with sections of human heart in the histo-immuno-fluorescence test described by BEACHEY et al, J. Exp. Med., Ed. 1977, 145, page 1469.

TABLE IV

| Immunization | | | Antibody titers | | | |
|---|---|---|---|---|---|---|
| Primary | Secondary (1) | Secondary (2) | at day 125 | | at day 225 | |
| J 0 et J 60 M-24 | J 115 SCB-7 | J 200 Oligo SCB-7 | Anti-SCB-7 | Anti M-24 | Anti-SCB-7 | Anti-M-24 |
| M-24 ( 10 ug) | SCB-7 (50 µg) | Oligo SCB-7 (50 µg) | < 160<br>< 160<br>< 160<br>< 160 | < 160<br>< 160<br>< 160<br>< 160 | 45,000<br>40,000<br>8,000<br>4,500 | 37,000<br>35,000<br>3,300<br>2,000 |
| M-24 (10 µg) | - | - | < 160 | < 160 | < 200 | < 200 |

EXAMPLE III :
A production of a secondary immune response protective
against diphteria by a synthetic octodecapeptide
containing a sequence of the "diphteria loop"

16 Swiss female mice received a primary immunization by diphteria anatoxine at the dosage of 0.3 Lf (1
microgram) administered sub-cutaneously in the 0.2 ml of
physiologically buffered aqueous solution. 30 days later,
8 mice did not receive anything and the other 8 mice
received the synthetic diphteria peptide SODP of formula:

$H_2N$-Ala-Ala-Cys-Ala-Gly-Asn-Arg-Val-Arg-

Arg-Ser-Val-Gly-Ser-Ser-Leu-Lys-Cys

The sera were taken up on day 40 (i.e. 40 days
later). the anti-diphteria antibody rate was measured by
the ELISA method as disclosed in the preceding examples in
relation to the streptococcic M protein. The biological
activity of these antibodies was evaluated by an in vitro
test of the inhibition of the toxicity of diphteria toxine. The detailed procedure of this test has been described in the following articles:
- MOEHRING T.J., MOEHRING J.M., KUCHLER R.J. and SOLOTO-
ROVSKY M., J. Exp. Med., 1967, 126, 407-427;
- BONVENTRE P.F. and IMHOFF J.G., J. Exp. Med., 1967,
1962, 1107;
- MIDDLEBROOK J.L. and DORLAND, B.R., Can. J. Microbiol.,
1977, 23, 175-189.

The assay was ran as follows :
A stable line fo monkey fibroblasts, the Vero
strain commercialized by Flow Laboratories, was used. The
assay is based on the property possessed by the diphteria
toxine to inhibit the proteic synthesis in these sensitive
cells; the synthesis activity of the cells was evaluated
by measuring the incorporation of a carbon 14-labelled-
aminoacid (C14 leucine).

a) Establishment of a cytoticity range
Variable dosages of toxine were added to a constant number of Vero cells cultured in a Hepes medium. The
dosage of toxine inhibiting 50 % of the normal $c^{14}$ leucine
incorporation by the cells within 16 hours was measured,
i.e. $5 \times 10^4$ ng/ml.

b) Study of the neutralizing action of sera

The sera to be tested at different dilutions as well as the negative and positive controls were incubated for half an hour with the diphteria toxine at the 50 % cytotoxic dose. The mixtures were then added to the culture medium of the cells ($10^6$ cells per culture well). One half hour later the cells were washed and brought back into a culture medium containing 14C-leucine and a further cultured fopr 16 hours. After removal of the leucine still present in the supernatant by appropriate washings, the radioactivity present in the cells was measured in a bêta counter. The percentage of protection was calculated using as a 100 % reference the number of disintegrations per minute (dpm) obtained in controls not treated by the toxine.

The results provided in table V show that after boosting with free SODP (that is neither conjugated nor polymerized), the rate of antibodies recognizing the diphteria toxine and capable of neutralizing its action has been strongly increased.

TABLE V

| Immunization | | Antibodies responses (J 40) Protection (%) | | | | |
|---|---|---|---|---|---|---|
| Primary J 1 | Secondary J 30 | ELISA | by serum diluted to: | | | |
| | | | 1/20 | 1/50 | 1/100 | 1/400 |
| Diphteria anatoxin 0,3 Lf (1 µg) | ( ( – ( SODP 100 µg ( | 500 20,000 | 5 55 | 0 45 | 0 40 | 0 30 |

EXAMPLE IV :
Production of an anti-sporozoite secondary response against Plasmodium knowlesi after a primary immunization by the same peptide however conjugated to tetanic anatoxine and a secondary immunization with the same glutaraldehyde-oligomerized peptide.

The peptide was described by GODSON et al (Nature, 1983, 305, 29) ; it comprises 26 aminoacids, including the 24 aminoacids of the repetitive tandem sequence present in the sequence of the surface antigen "circumsporozoite protein" of Plasmodium knowlesi, plus one tyrosine and one cystine. It was conjugated to tetanic toxine according to the method disclosed hereafter. It was oligomerized by the same technique as that disclosed in Exemple II with respect to the streptococcic peptide S-CB7.

For the coupling of the peptide to tetanic toxine the following procedure was used. 25 micrograms of the toxine dissolved in 2.6 ml of sodium bicarbonate 0.1 M were mixed with 21 micrograms of the peptide. After contact for one hour under stirring and at ambiant temperature, glutaraldehyde was added in two steps in order to obtain a solution having a final concentration of 0.1 % of the coupling agent. The contact was maintained in darkness and ambiant temperature during three days. The solution containing the conjugate formed was recovered and dialized against the PBS buffer. The purified conjugate obtained was used for the primary immunization under the conditions which follow :

Mice (6 per group) received a first sub-cutaneous injection of 100 micrograms of the peptide-tetanic anatoxine conjugate contained in 0.2 ml buffered physiological water. 28 days later they received a booster injection of 100 micrograms of either the free peptide or the oligomerized peptide. At day 35, the animals were bled and the anti-peptide antibody titer measured by the ELISA method under the conditions disclosed with respect of the dosage of the antibodies against S-CB7. The detection of the sporozoite and the evaluation of the antibody titers were obtained by using a solid phase-radioimmunoassay with $^{125}$I-labelled rabbit antibodies.

The results obtained are reported in the following Table :

TABLE VI

| Immunization | | Antibodies titers |
| Primary<br>J 1 | Secondary<br>J 28 | J 35<br><br>ELISA |
|---|---|---|
| Pep-tetanus<br>anatoxin<br><br>100 μg | Free peptide<br>100 μg | . 5,000 |
| | Polymerized peptide<br>100 μg | 38,000 |

The result of the radioimmunoassay shows that the antibodies obtained by the oligomerized peptide recognized the natural structure.

EXEMPLE V :

Production of a secondary immune response in men against the M protein of streptococcus type 24 after reimmunization by the S-CB7 peptide and by the oligomerized S-CB7

23 volunteers received in a primary immunization two intramuscular injections at a 15 day-interval of the purified M24 protein under a dosage of 200 micrograms with or without murabutide (N-acetyl-muramyl-L-alanyl-D-(alpha-n.butyl-ester)-glutamine . These products were administered in the form of a solution in physiologically-buffered aqueous solution. On day 45, 19 individuals received 200 micrograms of the free peptide, and on days 90 and 120, 200 and 400 micrograms respectively of the oligomerized peptide. The 4 remaining individuals received nothing more than the two first injections of the M protein.

The following observations were made : None of the individuals had antibodies on day 0 against the M24 protein or the S-CB7 peptide. On day 48, significant protecting antibody titers were obtained. On day 90, the rates of anti-M24 protein-antibodies had decreased. On day 140, the antibody-levels in the individuals who had received no secondary immunization were extremely low land the sera were no longer found to be protective. To the contrary, in the individuals who had been treated by the peptide and the polypeptide, the antibody levels were high one month after the last injection of the oligomer, particularly when the primary immunization had been carried out in the presence of murabutide.

The detection methods of the peptide and of the protein as well as the inhibition tests (carried out under the same experimental conditions as in Example II) showed that on day 45 the antibodies formed recognized the protein well, yet the peptide but little, and that the antibodies formed on day 140 had an anti-peptide specificity as well as a protein specificity. Moreover, the sera obtained on day 120 had as good a protection capability as those obtained on day 45.

These results thus clearly showed the boosting effect induced by the peptide alone on the immunization which had been evoked initially against the larger-sized protein.

EXAMPLE VI :

Vaccination with an oligomer of cellobiuronic acid against pneumococcic infections

Cellobiuronic acid is a disaccharide which forms the basic structural element of the specific polysaccharide of pneumococcus of type III. The oligomer was prepared from para-aminophenyl cellobiuronic acid (PAP-gamma-cellobiuronide).

The PAP-gamma-cellobiuronide (PM about 450) was dissolved in 0.1 M bicarbonate (1 mg/ml of bicarbonate solution). 2 microliters of 25 % glutaraldehyde were added to the solution and the mixture was left for 24 hours under stirring at the temperature of the laboratory. 4 microliters of glutaraldehyde were added again 48 hours later and the pH was adjusted to 7 with HCl.

Immunization tests were carried out on Swiss mice. They underwent a primary immunization with the specific natural polysaccharide of Pneumococcus III. A booster immunization with the above oligomer increased the protection previously acquired against Pneumococcus III.

Thus more generally, the invention relates to pharmaceutical compositions prepared under the above-indicated conditions, advantageously constituted by solutions, suspensions or injectable liposomes, containing a dose of at least one vaccinating synthetic haptene, particularly a peptide, or preferably an oligomer of this peptide, at a dosage adjusted to be effective in immunitarily non naive individuals. Preferably, these solutions, suspensions or liposome forms are prepared with an isotonic sterilized aqueous phase, preferably saline or glucosed. Preferably, the unit doses are of the order of 50 micrograms to 5 mg, advantageously from 100 micrograms to 1 mg.

The invention relates more particularly to such suspensions, solutions or liposome forms conditioned for use in booster immunization and adapted to be administered by intradermal, intramuscular or subcutaneous injections, or again by scarification.

It relates also to so-conditioned phrmaceutical compositions administrable orally, particularly to pharmaceutical compositions in which the peptide and/or oligomer is associated with pharmaceutically acceptable solid excipients or liquids, adapted for the constitution of oral forms.

Advantageously, the vaccinal compositions according to the invention contain in addition a vehicle, such as polyvinyl-pyrrolidone, facilitating the administration of the vaccine. In place of polyvinyl-pyrrolidone, there may be used any other type of adjuvant in the conventional sense given otherwise to this expression, that is to say a substance enabling the easier absorption of a drug principle or facilitating its action in the organism. They are well known to the man skilled in the art. By way of examples adjuvants of the latter type include for instance carboxymethylcellulose, aluminium hydroxide and phosphates.

It naturally goes also without saying that the doses which have also been indicated above by way of example may be used in the kits which have been considered above.

As concerns other haptenes likely to be used in the frame of the invention reference can also be made to the published European Application Nr. 83400525.8.

Preferred vaccine compositions as defined hereabove contain haptenes which have a molecular weight of less than 5000 daltons, preferably less than 3000 daltons or even less than 2000 daltons (or oligomers of said haptenes).

When the haptenes used in the compositions of the invention consist of peptide, protein or glycoprotein, they advantageously contain less than 50 aminoacyl residues, preferably less than 30, and possibly even less than 15 aminoacyl residues. When the haptenes used in the composition of the invention are formed of polysachharidic chains, they preferably contain less than 10 saccharidic units.

Needless to say that the composition according to the invention may also contain immunological adjuvants, particularly of the muramyl peptide type. Reference can be had to those muramyl peptides which are disclosed in the different patents which have been identified hereabove, all of which are incorporated herein by reference.

When reference in this disclosure and in the claims hereafter is made to immunological adjuvants associated with the haptenes or haptene-oligomers concerned, it is understood that this expression excludes covalent chemical couopling or conjugation of the muramylpeptides to the haptenes or haptene-oligomers. Further, when reference is made to the freedom of conjugation between the haptenes (or haptene-oligomers) and carrier molecules, it should be understood that this expression excludes more particular carrier molecules having molecular weights of about 20 000 daltons. Needless to say that, for instance, haptenes which may comprise a few aminoacyl residues or small peptide residues linked to one or two extremities of a sequence normally otherwise also present in the peptidic antigen from which said haptene is derived (or equivalent in their immunological properties to the last mentioned sequence) are not excluded from such definition. Such additional aminoacyl residues may be present because they participated in some way in the easiness of the chemical synthesis of the haptenic peptide. Needless to say that similar observations are to be made with respect to other types of haptenes.

Finally, it should be understood that when reference is made to the protective immunogenicity, reference is made to the results of protection which can be obtained not only in actual immunization assays in the hosts to be subjected to vaccination programs. Reference is also made to the standard tests for determining such protection capability, particularly in in vitro tests bringing into play either animals, cell cultures and their behaviors in the standard tests or specific reactions with determined components. Some of the preceding examples are representative of how such protective character can be determined. Obviously reference can also be made with test results in individuals or veterinary animals when they are practised. They can then be compared to those which are deemed representative of immunological protection in the relevant host when standard vaccination procedures are used (such as in vitro antibody titers measured on sera).

Finally the invention is also concerned with compositions of the type hereabove defined in which the haptene is part of a larger-sized hormone, under such circumstances in which the larger-sized hormone could be used for primary immunization and the haptene for secondary protective immunization (for instance in veterinary contraception). Such larger sized hormones should then be considered as equivalents of the "natural antigens" considered hereabove in relation to primary vaccination.

As is self-evident and as emerges already besides from the foregoing, the invention is in no way limited to those of its types of application and embodiments which have been more especially envisaged ; it encompasses thereof on the contrary all modifications.

CLAIMS

1. Vaccine composition against a pre-determined pathogenic agent, containing a synthetic haptene possessing itself an antigenic site characteristic of said pathogenic agent or an oligomer of this haptene, said haptene or haptene-oligomer being free of conjugation with a carrier molecule or with an adjuvant molecule, and said haptene or haptene-oligomer being conditioned at a dosage of haptene or haptene-oligomer suitable for the immunization of an immunitarily non-naive subject against said pathogenic agent, in association with a physiologically acceptable vehicule.

2. The vaccine composition of claim 1, for use in a subject who underwent primary vaccination and at a suitable time thereafter, said primary vaccination having been carried out with a composition effective to provide primary protection of said subject against said pathogenic agent.

3. The vaccine composition of claim 1 for use in a subject whose immunitary non-naiveness has been established by in vitro testing.

4. The vaccine composition of any of claims 1 to 3, which contains a haptene-oligomer and wherein said oligomer comprises from 2 to 10 monomeric units.

5. The vaccine composition of any one of claims 1 to 3 wherein the haptene has a molecular weight which does not exceed 5 000 daltons.

6. The vaccine composition of any one of claims 1 to 3 whose molecular weight is less than 3 000.

7. The vaccine composition of any one of claims 1 to 3 whose molecular weight is less than 2 000.

8. The vaccine composition of any of claims 5 to 7, which contains a oligomer of said haptene, comprising from 2 to 10 monomeric units.

9. The vaccine composition of any of claims 1 to 3 wherein said haptene is a peptide comprising less than 50 aminoacyl residues.

10. The vaccine composition of claim 9 wherein said peptide contains less than 30 aminoacyl residues.

11. The vaccine composition of claim 10 wherein said peptide contains less than 15 aminoacyl residues.

12. The vaccine composition of any of claims 9 to 11 which contains an oligomer of said haptene and wherein said oligomer comprises from 2 to 10 monomer units.

13. The composition of any one of claims 1 to 3 wherein said haptene is an oligosaccharide.

14. The composition of claim 13 wherein said haptene contains of from 1 up to 10 saccharidic groups.

15. The composition of any one of claims 1 to 14 which is devoid of any immunological adjuvant associated therewith.

16. The composition of any one of claims 1 to 14 which is associated with an immunological adjuvant.

17. The composition of claim 16 wherein said immunological adjuvant is a muramyl peptide.

18. The composition of any one of claims 1 to 17 which contains from 50 micrograms to 5 mg of said haptene or haptene-oligomer.

19. The composition of any one of claims 1 to 17 which contains from 100 micrograms to 1 mg of said haptene or haptene-oligomer.

20. The composition of anyone of claims 1, 9 or 12, wherein the synthetic haptene consists of a peptide bearing an antigenic site of the HBs antigene.

21. The composition of claim 20, wherein the peptide has the formula:

Asp - Tyr - Gln - Gly - Met - Leu - Pro - Val -
X - Pro - Leu - Ile - Pro - Gly - Ser - Y -
Thr - Thr - Ser - Thr - Gly - Z - X

in which

X is cystyl, aminobutyryl, alanyl or seryl,

Y is seryl or threonyl,

Z is prolyl or seryl.

22. The composition of any one of claims 1, 9, 18 or 19, wherein the haptene is constituted by a peptide comprising at the most 35 aminoacid residues and containing an antigenic site of M protein of Streptococcus pyogenes.

23. The composition of any one of claims 1, 2, 3, 18 or 19, wherein the haptene contains an antigenic site

of a pathogenic pneumococcus strain.

24. The composition according of claim 21, wherein the haptene is constituted by an oligomer of para-aminophenyl cellobiuronic acid.

25. A composition of matter in the form of a kit suitable for a cycle of vaccinations against a pre-determined pathogenic agent, which comprises, in combination:
- a dose of a vaccinating principle, particularly a natural vaccine selected from among those effective to confer a first protective immunization in a naive host, and
- at least one dose for at least one booster vaccination of a haptene or haptene-oligomer according to any one of claims 1 to 14 containing an antigenic site characteristic of the vaccinating principle for said first protective immunization, said haptene or haptene-oligomer being free of conjugation with a carrier molecule or with an adjuvant molecule, said haptene or haptene-oligomer being conditioned at a dosage of haptene or haptene-oligomer suitable for the immunization of an immunitarily non-naive subject against said pathogenic agent, in association with a physiologically acceptable vehicle.

26. A composition of matter in the form of a kit for at least one booster vaccination against a pre-determined pathogenic agent which contains, in combination:
- means for taking a sample of blood or of serum from the subject to be re-vaccinated;
- the elements or reagents, or both, suitable for the in vitro diagnosis of the immunitary non-naiveness of the host to be vaccinated against said pathogenic agent on said blood or serum sample;
- control biological blood or serum samples from immunitarily naive and, optionally, non-naive hosts or, alternatively, charts, or both;
- at least one dose-unit of a haptene or of an oligomer of said haptene according to any one of claims 1 to 14, said haptene or oligomer containing an antigenic site characteristic of the corresponding pathogenic agent or of a natural vaccine principle active against this pathogenic agent, said haptene or oligomer being conditioned in dosage unit form containing a dosage of haptene or hapten-

**0133833**

oligomer effective to maintain or reevoke a past protective immunization against said pathogenic agent.

27. The composition of matter of claim 26, wherein the elements and reagents necessary for carrying out an _in vitro_ diagnosis of the immunitary non-naiveness of the host on the blood or serum sample of the subject to be re-immunized against said pathogenic agent, comprise a microplate and the reagents necessary for measuring in a comparative manner the titers of antibodies against said pathogenic agent respectively contained in a blood or serum sample from the subject to be vaccinated and in a corresponding control biological sample from an immunitarily naive subject.

### European Patent Office

## EUROPEAN SEARCH REPORT

. Application number

EP 84 40 1612

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| P,X | WO-A-8 303 547 (BITTLE et al.)<br>* Page 27, lines 16-21; claim 22 *<br>--- | 1 | A 61 K 39/385<br>A 61 K 39/29<br>A 61 K 39/02<br>A 61 K 39/12 |
| X | BIOLOGICAL ABSTRACTS, vol. 58, 1st August 1974, page 1521, no. 14185, Philadelphia, USA; D.H. SCHMIDT et al.: "Persistence of hapten-antibody complexes in the circulation of immunized animals after a single intravenous injection of hapten" & J. EXP. MED. 139(2), 278-294, 1974<br>* Abstract *<br>--- | 1 | |
| D,A | NATURE, vol. 292, no. 5822, 30th July 1981, pages 457-459, Chesham, Bucks, GB; E.H. BEACHEY et al.: "Type-specific protective immunity evoked by synthetic peptide of Streptococcus pyogenes M protein"<br>* Page 457, right-hand column, lines 31-42 *<br>----- | 1 | **TECHNICAL FIELDS SEARCHED (Int. Cl.4)**<br><br>A 61 K |

The present search report has been drawn up for all claims

| Place of search THE HAGUE | Date of completion of the search 05-11-1984 | Examiner DAUKSCH H.J. |
|---|---|---|